# EUROPEAN PATENT SPECIFICATION

(11) **EP 2 765 955 B1**
(45) Date of publication and mention of the grant of the patent: **20.11.2019**
(21) Application number: 12840579.2
(22) Date of filing: 12.10.2012
(51) Int. Cl.: A61F 2/38

(54) **METHODS AND SYSTEMS FOR IDENTIFICATION, ASSESSMENT, MODELING, AND REPAIR OF ANATOMICAL DISPARITIES IN JOINT REPLACEMENT**
VERFAHREN UND SYSTEME ZUR IDENTIFZIERUNG, BEURTEILUNG, MODELLIERUNG UND REPARATUR VON ANATOMISCHEN UNGLEICHHEITEN BEI EINEM GELENKERSATZ
PROCÉDÉS ET SYSTÈMES POUR L'IDENTIFICATION, L'ÉVALUATION, LE MODELAGE ET LA RÉPARATION DE DISPARITÉS ANATOMIQUES DANS LE REMPLACEMENT D'ARTICULATION

(30) Priority: 14.10.2011 US 201161547349 P
(43) Date of publication of application: 20.08.2014
(73) Proprietor: ConforMIS, Inc., Billerica, MA 01821 (US)
(72) Inventor: BOJARSKI, Raymond, A., Attleboro, MA 02703 (US); FITZ, Wolfgang, Sherborn, MA 01770 (US)
(74) Representative: Grund, Martin
(86) International application number: PCT/US2012/059936
(87) International publication number: WO 2013/056036

(56) References cited:
- WO-A1-2004/043305
- WO-A1-2010/140036
- US-A- 4 936 862
- US-A- 4 936 862
- US-A1- 2010 274 534
- US-A1- 2011 071 645
- US-A1- 2011 087 332
- US-A1- 2011 087 332

## Description

### TECHNICAL FIELD

This application relates to improved systems and methods for deriving anatomical structures indirect anatomical measurements and/or comparison databases, as well as identifying abnormal, deformed, unusual, and/or undesirable anatomical structures, and related improvements in designing and/or selecting patient-adapted (e.g., patient-specific and/or patient-engineered) orthopedic implants and guide tools, as well as related methods, designs, and models.

### BACKGROUND

Due to the wide variation of what are considered "normal" anatomical features across the human population, it is often difficult to quantify and detect "abnormal" anatomical features, disparities, and/or other deformities in a given member of the population undergoing medical treatment, including joint replacement. Moreover, for patients with "abnormal" anatomical features, including anatomical disparities and/or deformity of their joints, as well as other anatomically incorrect and/or unusual anatomical structures, implants designed for "normal" joint replacement (such as, for example, implants and procedures to correct typical anatomical deficiencies) may be unsuitable, unstable, and/or suboptimal. Further, it can be difficult and/or time consuming to design an appropriate patient-specific joint replacement implant and surgical procedure for individual patients possessing such "abnormal" anatomy. Accordingly, there remains a need in the art for reliable systems and methods for determining when a given patient's anatomy is "abnormal," as well as for reliable systems and methods for assessing, designing, and/or selecting an appropriate joint replacement implant suitable for the repair and/or restoration of such "abnormal" or otherwise undesirable anatomy. Repairing or restoring a diseased, damaged, or deformed joint to original healthy bone structure is one desired goal in surgical joint procedures.

### SUMMARY

According to the invention, a method of making an implant component for a joint of a patient is disclosed.

The method according to the invention includes obtaining an anatomical dimension associated with the patient. The anatomical dimension is used along with an anatomical relationship correlation to derive information regarding an anatomical feature of the joint. The anatomical feature is also measured, and the measured information is compared to the derived information regarding the anatomical feature. The comparison of the measured information to the derived information is then used in designing the implant component.

### BRIEF DESCRIPTION OF THE DRAWINGS

Objects, aspects, features, and advantages of various embodiments will become more apparent and may be better understood by referring to the following description, taken in conjunction with the accompanying drawings, in which:
**FIG. 1** is an image of a perspective view from the lateral side of a distal femur showing medial and lateral condyles;
**FIG. 2** depicts the image of **FIG.1**, with the medial condyle information hidden or otherwise removed;
**FIG. 3** shows the outer surface of the lateral condyle from **FIG. 2**, with the remainder of the condlye hidden or otherwise removed;
**FIG. 4** shows a continuous surface profile created along the lateral condyle surface of **FIG. 3**;
**FIG. 5** depicts a final condylar J-Curve profile for the lateral condyle, obtained through smoothing and/or other manipulation of the continuous surface profile of **FIG. 4**;
**FIG. 6** depicts an exemplary set of measurements that can be taken of a distal femur;
**FIG. 7** depicts a width measurement **A** of a distal femur taken at the widest section of the load bearing portion of the bone;
**FIG. 8** is a graphic representation of one set of generated relationships between a femur width and the anterior and posterior radii of a medial femoral condyle of the femur;
**FIG. 9** is a graphic representation of one set of generated relationships between a femur width and the anterior and posterior radii of a lateral femoral condyle of the femur;
**FIG. 10** depicts a distal femur showing an exemplary placement of anterior and posterior radii for deriving a surface profile of a condyle;
**FIG. 11** depicts a distal femur showing medial and lateral J-curves measured from the condyle surface profiles;
**FIG. 12** depicts an anterior radius of the lateral J-curve of **FIG. 11**;
**FIG. 13** depicts a posterior radius of the lateral J-curve of **FIG. 11**;
**FIG. 14** is one example of a distal femur showing medial and lateral J-curves derived from femoral width measurements;
**FIG. 15** depicts an anterior radius of the lateral J-curve of **FIG. 14**;
**FIG. 16** depicts a posterior radius of the lateral J-curve of **FIG. 14**;
**FIG. 17** is a set of exemplary contour ratios determined experimentally from a population of individuals demonstrating the relationships between the anterior and posterior radii of the condyles of the femur;
**FIG. 18** is a graphic representation of one set of generated relationships between a patient's height and a width of the patient's femur;
**FIG. 19** is a graphic representation of one set of generated relationships between the width of a patient's femur and the widths of the patient's medial and lateral condyles;
**FIG. 20** is a graphic representation of one set of generated relationships between the width of a patient's femur and the depths of the patient's medial and lateral condyles;
**FIG. 21** is a graphic representation of one set of generated relationships between the width of a patient's femur and the depths of the patient's medial and lateral tibial plateaus;
**FIG. 22** is a graphic representation of one set of generated relationships between the width of a patient's femur and the length and with of the patient's patella;
**FIG. 23** depicts a graphic representation of one set of gender-neutral correlations between the width of a patient's femur and the lengths of the patient's medial and lateral condyles, with an associated relationship equation and experimental results including a standard deviation and standard error of the mean;
**FIG. 24** depicts a graphic representation of one set of gender-specific correlations between the width of a patient's femur and the lengths of the patient's medial and lateral condyles, with an associated relationship equation and experimental results including a standard deviation and standard error of the mean;
**FIG. 25** depicts a graphic representation of an alternate set of gender-specific correlations between the width of a patient's femur and the lengths of the patient's medial and lateral condyles, with an associated relationship equation and experimental results including a standard deviation and standard error of the mean;
**FIG. 26** depicts a graphic representation of a set of gender-neutral correlations between the width of a patient's femur and the widths of the patient's medial and lateral condyles, with an associated relationship equation and experimental results including a standard deviation and standard error of the mean;
**FIG. 27** depicts a graphic representation of a set of gender-specific correlations between the width of a patient's femur and the widths of the patient's medial and lateral condyles, with an associated relationship equation and experimental results including a standard deviation and standard error of the mean;
**FIG. 28** depicts a graphic representation of another set of gender-specific correlations between the width of a patient's femur and the widths of the patient's medial and lateral condyles, with an associated relationship equation and experimental results including a standard deviation and standard error of the mean; and
**FIG. 29** depicts a graphic representation of a set of gender-neutral and gender-specific correlations between the width of a patient's femur and the width of the patient's femoral notch, with an associated relationship equation and experimental results including a standard deviation and standard error of the mean.

Additional figure descriptions are included in the text below. Unless otherwise denoted in the description for each figure, "M" and "L" in certain figures indicate medial and lateral sides of the view, respectively; "A" and "P" in certain figures indicate anterior and posterior sides of the view, respectively; and "S" and "I" in certain figures indicate superior and inferior sides of the view, respectively.

### DETAILED DESCRIPTION

In this application, the use of the term "including," as well as other forms, such as "includes" and "included," is not limiting. Also, terms such as "element" or "component" encompass both elements and components comprising one unit and elements and components that comprise more than one subunit, unless specifically stated otherwise. In addition, the use of the term "portion" may include part of a moiety or the entire moiety.

Additionally, in this application, use of the terms "implant" and "implant component" encompass both an implant or component that is one of multiple implants or components making up a single implanted structure and an implant or component that constitutes the entire implanted structure. Further, an "implant system" can include one or more implant components and, optionally, one or more related surgical tools.

Various embodiments herein describe systems and methods for deriving and/or assessing the anatomy, including the articular surfaces, of one or more patients. Further embodiments describe systems and methods for designing or selecting one or more implant designs, surgical tools, surgical procedures, and/or treatments for addressing abnormal and/or deformed joint structures. Various embodiments contemplate the use of dimensional and measurement databases and/or libraries for assisting and verifying implant design rationale and specifications, as well as the alignment and estimation of anatomical structures and surfaces (including, but not limited to, approximation of J-curvatures in healthy, deformed, and/or abnormal bone) using computer programs, anatomical relationships, and/or mathematical algorithms to plot anatomical bone structure in deformed or abnormal bone as well as derive original (i.e., pre-disease) or desired bone structure from patient diseased bone and/or non-patient anatomical information. The present systems and methods also facilitate the validation, verification and/or quality review of proposed surgical plans using dimensional and or other measurements of anatomical features to derive anatomically accurate "estimates" of deformed or abnormal joint structures (including such structures in healthy and/or damaged conditions) using image data including CT image data, etc. Various embodiments facilitate the development and use of bone preserving and anatomical preservation techniques in the treatment of healthy and/or diseased joints and ligamentous structures. In addition, the disclosed systems and methods increase the accuracy and reliability of surgical procedures and provide for quantifiable methods for improving surgical treatments, selection and/or design specifications of implant systems, and ultimately patient outcomes.

It is often desirable for a joint replacement implant to attempt to replicate the normal motion of the joint it is destined to replace. For example, where a joint has been damaged by trauma or otherwise injured through high stress/forces and/or repetitive loading activities such as sports or manual labor, the joint replacement components are often designed to replicate the joint, which can include (if necessary) reconstructing the existing joint in a healthy condition (e.g., a damaged joint is virtually or physically reconstructed, and then an implant is designed to replicate the reconstructed joint such as a knee, etc.) However, for patients whose joints have undergone extensive degeneration and/or remodeling (or other wear) over a period of time, or for individuals born with deformed anatomy, it may be difficult to virtually reconstruct the healthy joint condition, or to even identify the existence and/or extent of degeneration. These difficulties are often exacerbated by the wide variety of anatomical variation seen across healthy populations. In many instances, the direct use of image data in the design and/or selection of a patient's implant may be difficult where the image data (i.e., from CT images, X-Ray, MRI, orthoscopic image, etc.) depicts joint structures having significant bone loss and/or other structural instabilities, including valgus or varus misalignments, rotational issues, undesirable tissue tension and/or laxity, an overabundance of osteophytes, voids, etc.

Various embodiments include systems and methods for directly measuring various anatomical features of a patient's joint, as well as deriving and/or estimating various anatomical features of the patient's joint through indirect measurement and/or derivations. The various direct and "derived" measurements can be compared and/or contrasted, if desired, and areas of disparity and/or deviation are identified, assessed and addressed. In this manner, diseased, damaged, unusual or unexpected anatomical features can be identified, and for various anatomical features it may be possible to estimate and/or reconstruct the feature in a healthy or less diseased or damaged state.

Various articles and publications have discussed and/or inferred numerous relationships and/or correlations between various anatomical features of a given individual as well as among individuals of specific population groups (i.e., age, weight, gender, race, etc.), including "Knee Morphology as a Guide to Knee Replacement," by Mensch and Amstutz, Clin Orthop Relat Res., 1975 Oct; (112):231-41, "Differences Between the Sexes in the Anatomy of the Anterior Condyle of the Knee," by Fehring, Odum, Hughes, Springer and Beaver, J Bone Surg Am. 2009; 91:2335-41, "Superio-Inferior Relationship Between Medial and Lateral Femoral Condyles," by Singh, Aggarwal, Singh and Sapra, J Anat Soc India, Vol. 50, No. 2 (2001-07 - 2001-12), "Mathematical Reconstruction of Human Femoral Condlyes," by van den Heever, Scheffer, Erasmus and Dillon, Journal of Biomech Engr, June 2011, Vol. 133, "Method for Selection of Femoral Component in Total Knee Arthroplasty (tka)," by van den Heever, Scheffer, Erasmus and Dillon, Australas Phys Eng Sci Med (2011) 34:23-30, "Dimensions of the Knee. Radiographic and Autopsy Study of Sizes Required by a Knee Prosthesis," by Seedhom, Longton, Wright, et al., Ann Rheum Dis 1972 31: 54-58, the disclosures of which are each incorporated herein in their entireties by reference.

In one embodiment, an anatomical feature is measured directly from an image of the patient's joint, while information regarding the structure of the same anatomical feature is derived (i.e., indirectly derived from measurements of other and/or adjacent anatomical structures) from other anatomical measurements, which can be utilized in combination with non patient-specific data such as databases, mathematical formulae, geometric relationship data, statistical models, shape models and/or other information sources. The measured anatomical feature is then compared to the derived anatomical feature, and disparities are identified for further action and/or processing. Where there is little disparity between the derived anatomical feature and the measured anatomical feature, such information may indicate that the actual patient anatomy approximates "normal" or acceptable anatomy (or otherwise matches the comparison database information), and thus the measured anatomy (and/or the derived anatomy) can be identified as suitable for direct design of an appropriate joint-replacement implant and/or surgical implantation procedure. Where there is significant disparity between the derived anatomical feature and the measured anatomical feature, however, such information may indicate that the patient anatomy is unusual and/or otherwise unsuitable for direct design of an appropriate joint-replacement implant and/or surgical implantation procedure, possibly necessitating manipulation and/or reassessment of the anatomical and/or image data prior to implant design and/or selection.

In various embodiments, one or more of the derived anatomical feature measurements may be utilized in place of actual feature measurements for the design and/or selection of an implant if relevant criteria indicate some disparity or other concern regarding the accuracy and/or suitability of the actual feature measurement(s). Similarly, one or more of the derived anatomical feature measurements may be utilized to modify or otherwise alter actual feature measurements for the design and/or selection of an implant if relevant criteria indicate disparity or other concern regarding the accuracy and/or suitability of the actual feature measurement(s).

In various embodiments, the systems and methods can include the creation of "derived" implant design(s) and/or surgical procedure(s) steps appropriate to the derived anatomical features, and the creation of "normal" implant designs and/or surgical procedure steps designed and/or selected using the actual measured features of the joint. The implants and/or surgical methods can be compared, and the disparities and/or differences between the "derived" design/surgical plan and the "normal" design/surgical plan can be identified, assessed, quantified and/or addressed. Where there is little disparity between the proposed design and the normal design for the patient's implant, such information may indicate that the patient anatomy approximates "normal" or otherwise desirable anatomy, and thus a normal joint-replacement implant may be suitable for the patient. In a similar manner, if there is little disparity between the proposed surgical procedure steps and the normal surgical procedure steps, such information may indicate that the patient anatomy approximates "normal" anatomy, and thus a normal surgical procedure for the joint-replacement may be suitable for the patient.

In the event that significant disparities exist between the proposed design and/or surgical plan and the normal design and/or surgical plan, such disparities may indicate that the patient anatomy is "abnormal" or otherwise deformed and a "normal" implant and/or surgical plan may not be appropriate and/or an optimal solution for the patient. In such a case, the method may include instructions to "flag" the implant designs (and/or procedure steps) or provide both design/procedure approaches (and results) and identify differences, as well as give recommendations.

By deriving information regarding two anatomical features, two potential implants, two sets of surgical jigs/instruments and/or two surgical plans from unique patient-specific data using two different approaches and methodologies, and then comparing and/or assessing the relative merits of each, the present systems and methods can desirably identify "abnormal" or otherwise undesirable anatomy. Moreover, where the two approaches identify similar designs and surgical steps, the system and methods may confirm the appropriateness of the measurements, designs and surgical procedures for the particular patient.

Various image sources for the bone anatomy information of a patient can be utilized, including the use of image data in hard copy as well as 2 dimensional or 3 dimensional electronic image formats. In addition, a wide variety of data manipulation and display programs can be utilized to view and/or manipulate the data, including computer aided design (CAD) programs. Desirably, the image data will be obtained from non-invasive image sources, to allow for the design/selection and manufacturing of the implant and surgical alignment tools prior to surgery.

Moreover, the data could come from single or multiple 2D and/or 3D image sources, and utilized in their native formats or be combined to create a 3D representation of the biological structure. Alternatively, the images can be combined to form a 3D data set, from which the 3D representation of the biological structure can be derived directly using a 3D segmentation technique, for example an active surface or active shape model algorithm or other model based or surface fitting algorithm.

Optionally, the 3D representation of the biological structure can be generated or manipulated, for example, smoothed or corrected, for example, by employing a 3D polygon surface, a subdivision surface or parametric surface, for example, a non-uniform rational B-spline (NURBS) surface. For a description of various parametric surface representations see, for example Foley, J. D. et al., Computer Graphics: Principles and Practice in C; Addison-Wesley, 2nd edition (1995). Various methods are available for creating a parametric surface. For example, the 3D representation can be converted directly into a parametric surface, for example, by connecting data points to create a surface of polygons and applying rules for polygon curvatures, surface curvatures, and other features. Alternatively, a parametric surface can be best-fit to the 3D representation, for example, using publicly available software such as Geomagic® software (Research Triangle Park, N.C.).

If desired, the data could be presented as part of a model, for example, a patient-specific virtual model that includes the biological feature of interest. Optionally, the data associated with one or more biological features can be transferred to one or more resection cuts, drill holes, guide tools, and/or implant components, which also can be included as part of the same model or in a different model. The virtual model(s) can be used to generate one or more patient-adapted guide tools and/or implant components for surgical use, for example, using CAD software and/or one or more of the several manufacturing techniques described below, optionally in conjunction with computer-aided manufacturing (CAM) software.

In various embodiments, the systems and methods include obtaining image data of a patient's anatomy in hard copy or in electronic form, obtaining anatomical data on the same or similar anatomical structures through two different procedural/derivation paths (i.e., obtaining direct feature data through direct measurement of an anatomical feature and deriving data about the same feature through indirect measurement and data manipulation), and comparing the directly measured and derived anatomical data to determine if discrepancies in the anatomic data exist. Discrepancies can be due to a wide variety of factors, including (1) the existence of unusual, degenerated and/or deteriorated anatomy, (2) incorrect or inaccurate measurement of the direct anatomical features and/or the indirect anatomical features, and/or (3) inaccurate or incorrect relationship data between the indirect measurement and the anatomical feature(s) of interest. If desired, various verification and/or confirmation steps can be included and/or incorporated into alternate embodiments to minimize measurement errors and/or to ensure proper selection of relationship data and/or database members.

In various embodiments, the systems and methods described herein could include the use of various anatomical relationship correlations, including those described herein as well as others determined experimentally and/or obtained from other sources, to create and/or design/develop implants, surgical tools and/or procedures, including various pre-designed and/or pre-manufactured implant libraries as well as associated libraries of surgical tools and procedures. The virtual library could be queried and one or more virtual designs chosen and further modified, if desired, for subsequent manufacturing and patient implantation of the physical implant. Such manufacturing could include "just-in-time" type manufacturing and inventory methods.

In various other embodiments, the library could include a physical inventory of a number of implants of different shapes and/or sizes designed, at least in part, using the various system and methods described herein. Such physical implants could include stockpiles of various pre-determined sizes and shapes of implants, which could be designed using the various systems and methods described herein, including scaling of various implant shapes and/or sizes. The library could optionally include one or more series of pre-manufactured implant "blanks" having various pre-determined dimensions and/or shapes, with the individual blank or blanks selected for further processing and/or modification to closely approximate the chosen virtual design for ultimate implantation into the patient.

While various embodiments are described herein in connection with the assessment of condylar articular surface features, the present disclosure would have varying utility with respect to many anatomical features, including, for example, femoral widths at various levels, lateral and medical condyle widths, lateral or medial condyle depths, femoral notch shape and dimensions, patellar shapes and sizes, tibia medial and lateral widths, tibia medial and lateral depths, tibial plateau shapes and dimensions. Furthermore, various embodiments described herein may facilitate design and/or selection of knee implant components and surgical tools and procedures configured to preserve one or both natural ligaments of the knee (anterior cruciate ligament and posterior cruciate ligament) and/or to substitute stabilizing features for the cruciate ligaments. For example, cruciate substitution embodiments may include, for example, an intercondylar housing (sometimes referred to as a "box"), a receptacle for a tibial post or projection, and/or one or more intercondylar bars. And design aspects (e.g., size, shape, location) of such features may be based, at least in part, on derived information regarding anatomical features (e.g., femoral notch width), as discussed further below.

In a typical patient-specific design of a joint-replacement implant and surgical procedure, various anatomical measurements from the patient are obtained, and that information may then generally be copied to design a joint-replacement implant and/or surgical procedure. For example, the medial and lateral J-curves from the patient's medial and lateral condyles of a femur may be assessed and quantified, and this information directly used (and potentially filtered and/or modified in some manner, if desired) to design and/or select a joint replacement having similar J-curves. Similar measurements may be taken for other anatomical features (e.g., coronal curvatures of the condyles, the shape of the trochlear groove and/or notch, etc.), and modified if necessary or desired, and then utilized to directly design and/or select surfaces and/or other features of the joint replacement implant, as well as surgical tools and surgical steps for preparing the joint and implanting the device.

However, where the patient's native anatomy is abnormal in some manner and/or has undergone significant degeneration and/or other remodeling (such as where significant remodeling and/or loss of bone on an articulating surface of the joint has occurred prior to acquisition of the image data), the native anatomy may be too deformed and/or degraded to provide accurate and/or useful data for approximating an optimal joint replacement implant and/or surgical procedure. In some situations, the abnormality of the anatomy may be a lifetime deformity or may be undetectable, easily ignored, and/or discounted, and thus, use of such information can result in an improper or suboptimal implant for use with the patient.

Some embodiments of the present application can include the derivation and/or estimation of the patient's joint anatomy, including, for example, the features of the J-curvatures of the medial and lateral condyles of the femur, utilizing measured and/or derived anatomical data from one or more sources that are less prone to significant structural degeneration and/or other remodeling (i.e., "indirect" measurements of the relevant anatomy). Using such data and approaches, a designer can approximate the healthy features of the joint (absent the deformities and/or abnormalities), allowing an appropriate implant and/or surgical approach to be designed and/or selected. If desired, the derived and/or estimated "healthy" features of the joint can be compared to the existing joint measurements, and significant differences can be identified and highlighted to the designer. In a similar manner, the derived and/or estimated implant, surgical procedures, and/or surgical tools/jigs can be compared to those implants, tools, and procedures derived using native joint measurements, and significant differences can be identified and highlighted to the designer. Where significant differences do not exist, such information may be useful as an indication that the anatomy has not experienced significant disease or degeneration or is otherwise of acceptable quality and/or values, and thus, may be suitable for direct use in designing and/or selecting a surgical implant, tool/jig, and/or surgical procedure.

Moreover, even where the patient may appear to have "healthy" or otherwise functional joint anatomy from direct visualization and/or measurement of the anatomy, a joint replacement implant designed and/or selected for use with the "healthy" joint might benefit from various structural and/or dimensional modifications. For instance, the healthy joint may have inherent structural flaws and/or performance limitations or constraints never noticed or identified by the designer, manufacturer, patient, and/or the physician. The comparison and/or incorporation of derived anatomical features and/or associated implant/tool/jig designs and surgical procedure plans, ether alone or in combination with directly measured anatomical features, would potentially result in an improved design and/or selection of a joint replacement implant, with potentially better patient outcomes.

In some embodiments, existing patient information can be obtained from various sources. For example, such information can come from measurements of a target joint's anatomical structures. By way of example, if the target joint is a knee, such information may come from measurements of features of the femur, tibia, and/or patella. The information may include general joint dimensions, as well as any number of biomechanical or kinematic parameters, as described in the foregoing sections and as known in the art. In this application, use of the terms "dimension(s)" and "anatomical dimension(s)" is intended to broadly include, but not be limited to, attributes such as height, width, length, depth, slope, curvature, radius of curvature, and various other measurements as well. In some embodiments, existing patient information can include, for example, information regarding the patient's femoral/tibial/patellar shape, length, and/or width; condyle dimensions, features, and/or slopes; angles (e.g., trochlear angle, Q angle); trochlear characteristics; tibial characteristics; tibial tuberosity; medial/lateral slopes; tibial spine height; coronal curvatures; and/or sagittal curvatures. The information can also include mechanical axes, epicondylar axes, and/or other anatomical and biomechanical axes or angles. Existing patient information may further include information from the patient's contralateral joint (e.g., the opposing knee, hip, or shoulder joint) and/or information regarding adjacent joint structures. Additional information collected can include height, age, body weight, race, gender, activity level, health conditions, other disease or medical conditions, etc. Patient outcomes post-surgery may also be collected in the database, if desired. Moreover, weighting parameters can be assigned to various measurements or series of measurements (or other collected or derived information), as well as to one or more joint surfaces, including opposing joint surfaces.

Certain embodiments may further include utilization of various of the collected and/or derived patient-specific information, as well as any optional weighting parameters, to query select data and/or databases and identify one or more "anatomical relationship correlations" from one or more reference databases (or choose pre-existing anatomical relationship correlations, if desired), comparing features from the subject to related patient anatomical structures (as well as structures of other individuals in the database), and optionally creating a comparison or "weighting score" to evaluate and display the results of the various comparisons (relative to individual feature comparisons and/or an overall composite score for the comparison of each subject). The databases can comprise information from various sources, including cadaveric data, imaging, biomechanical or kinematic data, historic data, and/or data regarding previous knee implant cases from various manufacturers, including existing patient-specific case data. Such data can be specific to gender, age, weight, health, size, etc., or can be selected based on weighting (as previously described) or other criteria. The correlations can include any number of factors, including comparisons regarding patient outcomes as compared to anatomical features (actual and/or derived) and/or implant features (actual and/or derived) and surgical plans (actual and/or derived), or various combinations thereof.

In various embodiments, the library/database can comprise various databases of dimensional and measurement findings from one or more individuals of a specific or of a general population. The individuals may be healthy or diseased or combinations thereof. The database can comprise information stored in either or both a digital and hard copy format, and can be used as a reference for identifying various anatomical relationships as well as verifying dimensions of an abnormal patient and the design specifications of the implant. If desired, the results of various surgical procedures and/or implant designs can be cataloged and/or incorporated into the database, including supplementing and/or improving the database with future surgical interventions and/or alterations to various implant design features as such become available.

Various embodiments then manually and/or automatically select one or more anatomic shapes or features or other information, which can be derived from a list of "anatomical relationship correlations" of the patient's anatomy as well as from one or more of the databases and/or matching subjects, to calculate or create one or more "derived anatomical features," which can subsequently be compared to the measured patient-specific data, if desired.

Depending upon the outcomes of the various comparisons of measurements or sets of measurement, the system may choose to maintain the measured (actual) data at its current measured value. Alternatively, the method may utilize the derived feature data to normalize, "smooth," or otherwise modify the measured patient-specific data, which can desirably correct or normalize the patient-specific data and potentially correct the patient-specific data for inherent deformities like osteophytes, voids, bone and tissue deterioration and/or degradation, axis deformity, and/or cartilage degradation. As another alternative, the method may utilize the derived feature data to replace some or all of the measured data. Alternative embodiments can include the use of various combinations of measured, modified, and/or replaced anatomical information in the design and/or selection of an appropriate implant, as well as in the creation of a desired anatomical model.

The "derived anatomic feature" data may comprise the features from one or more subjects, or may comprise a composite anatomy derived from such shapes and/or subjects (which may also be identified and/or derived utilizing a weighting score, if desired) from one or more databases and/or libraries.

In various embodiments, the anatomical measurements (from which the derived measurements are calculated or otherwise obtained) are desirably taken from anatomic features that are unlikely to become significantly deformed, diseased, and/or otherwise damaged as part of the patient's degenerative condition, or that are otherwise identified as reliable or "more likely to be reliable" than a comparable direct measurement data of the relevant feature(s). For example, where the medial and/or lateral condyles of a patient are likely to become worn and/or significantly remodeled over the patient's lifetime, the width of the patient's femur will generally remain constant for most (or significant portions) of the patient's life. In such a situation, the width information could be used to estimate condyle surface profile, even where the patient's native condyles have been significantly worn.

Various embodiments herein include the use of calibration phantoms or other measuring devices and methodologies known in the art to ensure accurate measurement of anatomical features from images and electronic data. In addition, it may be desirous for a physician to utilize standard imaging techniques and known alignment methodologies such as, for example, a modified posteroanterior tunnel view and/or a true lateral view of a patient's joint, to ensure that misalignment of the anatomical structures relative to the imaging equipment does not significantly impact the accuracy and reliability of the various systems and methods described herein.

In various embodiments, the data is from a single subject while, in other embodiments, the data is from two or more subjects. In some embodiments, the methods further comprise compiling multiple databases from each database where the data points are collected from a single individual and the data points for each single individual are associated with one or more relevant data attributes. In any of the methods described herein, the quantitative information can be, for example, bone mineral density or density of selected soft-tissues or organs. Alternatively or additionally, the quantitative information is information on the morphology of a structure, for example information on the two-dimensional arrangement of individual components forming said structure or information on the three dimensional arrangement of individual components. In any of the methods described herein, the structure can be bone and the information can be, for example, information on trabecular thickness, trabecular spacing, and/or estimates of the two- or three-dimensional architecture of the trabecular network or as described in any of the measurements described above.

The various embodiments further provide a convenient source of comparative data for the assessment of a given patient's anatomical data. Currently, if a patient's image data appears to be abnormal and/or difficult to reconstruct, the patient may be forced to undergo imaging of the contralateral joint structure, as a source of data to assist with reconstruction of the damaged structure. This additional imaging can involve significant expense, patient and/or physician inconvenience, and added radiation exposure. By deriving anatomical features from structures already imaged, various embodiments described herein can obviate the need for imaging of the contralateral joint structure. Various embodiments described herein may still additionally or alternatively obtain information from imaging of the contralateral joint. Further, in instances where native anatomical data is of poor or insufficient quality (e.g., due to poor radiographic equipment and/or limited availability of imaging equipment, interference from image artifacts and/or unavailability of 3-dimensional and/or proper 2-dimensional bi-planar imaging equipment), the derivation of accurate and/or relevant anatomical structures using such limited information can be accomplished.

Desirably, the various systems and methods described herein will facilitate the design and/or selection of implant and surgical procedures that optimize a patient's recovery and maximize performance of their joint replacement implants. With incorporation of optimal J-curvatures on the articular surfaces of the implant, the knee replacement implant will desirably address mid-flexion instability issues common with currently-available implants, and thereby maintain stability of the knee throughout the entire range of motion of the implant.

Various embodiments include the creation of surgical tools (e.g., measuring and cutting jigs and/or other instruments) and surgical plans utilizing the various techniques described herein. Specific information and features regarding these various surgical tools and plans can be included in relevant databases, and queried to determine correlations between the various features and relative success and/or failure of relevant patient outcomes. These results can be utilized to identify potential concerns, and possibly lead to further modification of the patient's derived anatomy and/or proposed implant and/or surgical procedure designs. Similarly, results can be utilized to identify positive correlations to confirm a patient's derived anatomy and/or proposed implant and/or surgical procedure designs.

In various embodiments, the methods and systems can be incorporated into an automated or semi-automated design and/or selection algorithmn, computer program, and/or other system used to design implant components and/or surgical tools. If desired, the system could automatically check for anatomical discrepancies and/or other variations, could automatically or with user-approved intervention utilize such derived information in modeling and design/selection of implant components (if desired), and/or notify the user and/or correct the disparity using measured and/or derived anatomical data, or combinations thereof. If desired, various embodiments can incorporate and display both the measured anatomic features as well as the derived anatomic features. Furthermore, the information can be encrypted and/or transmitted remotely for further processing and/or review in any of the methods described herein.

### DERIVATION OF CONDYLAR SURFACES RELATIVE TO FEMORAL WIDTH

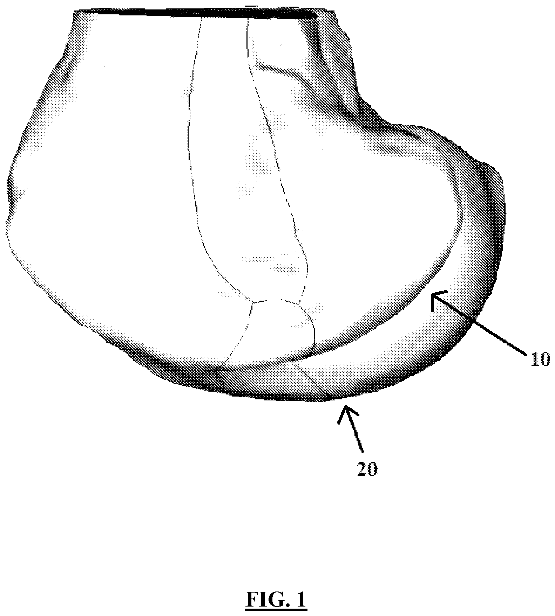

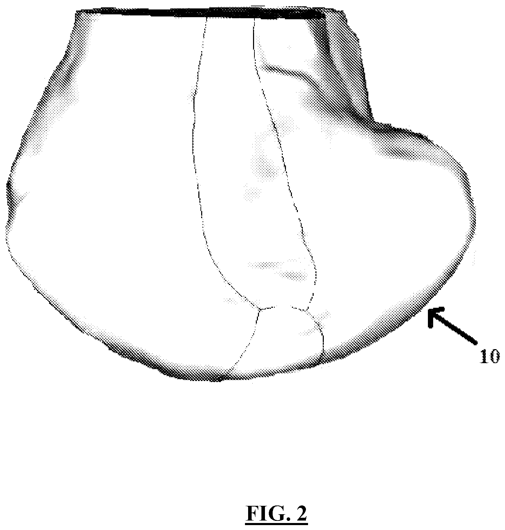

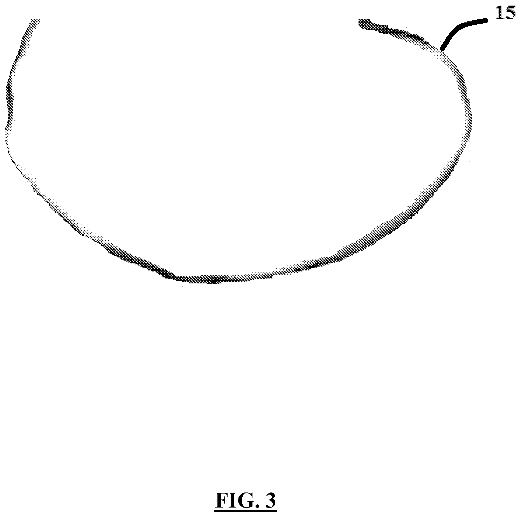

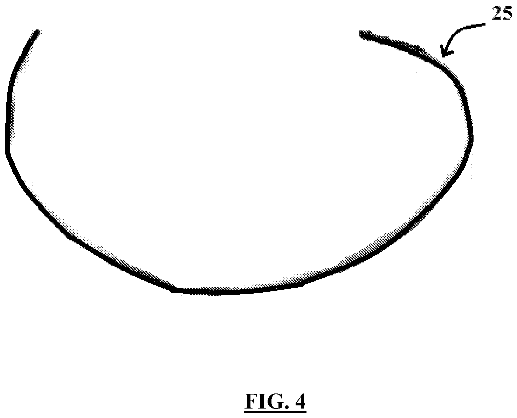

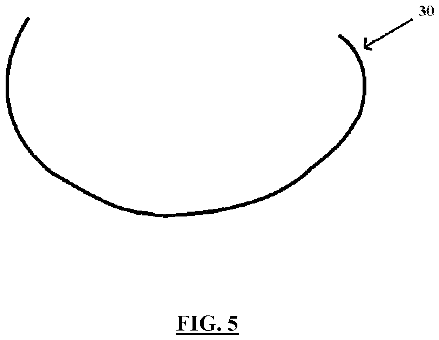

In a first exemplary embodiment, a "standard" derivation of the condylar surfaces of a patient's femur can be obtained directly from one or more images of the patient's native condyle. For example, a designer or automated program can begin with an electronic image of a patient's native femur. FIG. 1 depicts a side perspective view of the distal end of a femur, showing a lateral condyle 10 and a medial condyle 20. FIG. 2 depicts the electronic image showing only the lateral condyle 10, with the medial condyle information hidden or otherwise removed. In FIG. 3, the outer surface 15 of the lateral condyle from FIG. 2 has been highlighted or otherwise selected for further manipulation, with the remainder of the surface hidden or otherwise removed. FIG. 4 depicts the creation of a continuous surface profile 25 substantially following the lateral condyle surface of FIG. 3, which may be created by following the image surface information in combination with a smoothing and/or filtering operation (if desired). This surface profile 25 can be smoothed or otherwise manipulated, as desired, to create a final estimated and/or derived surface 30 for the lateral condyle, as best seen in FIG. 5. A similar approach and process can be utilized to derive a J-curvature for the medial condylar surface (not shown).

In a concurrent analysis (or before, after, or in place of the direct surface measurement, as desired), one or more relevant anatomical measurements of the patient's joint can be utilized to derive the same or similar anatomic features of the joint and/or its articulating surface indirectly. Desirably, these anatomical measurements have a relationship to various aspects of the articulating features of the joint such that the anatomical measurement can be utilized to derive various other joint measurements. More particularly, the measurements will desirably be taken from joint features that are not degenerated and/or diseased, and thus the measurements and relationships can be utilized to calculate various anatomical features of the joint in a non-diseased, pre-degeneration, and/or otherwise healthier or better-functioning state.

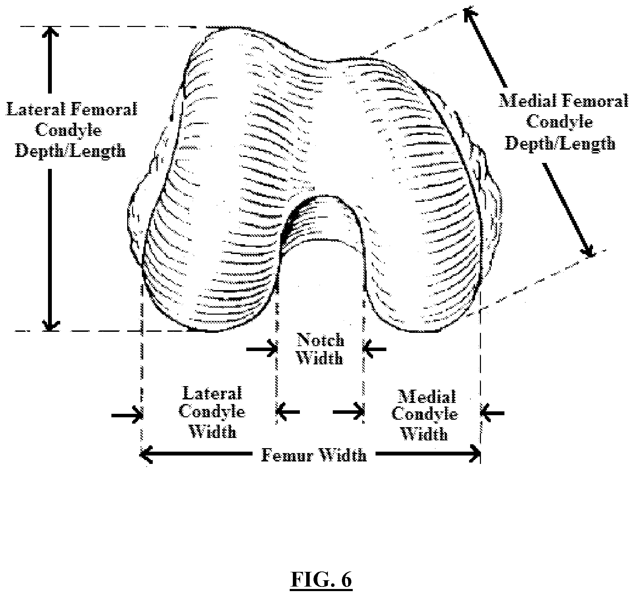

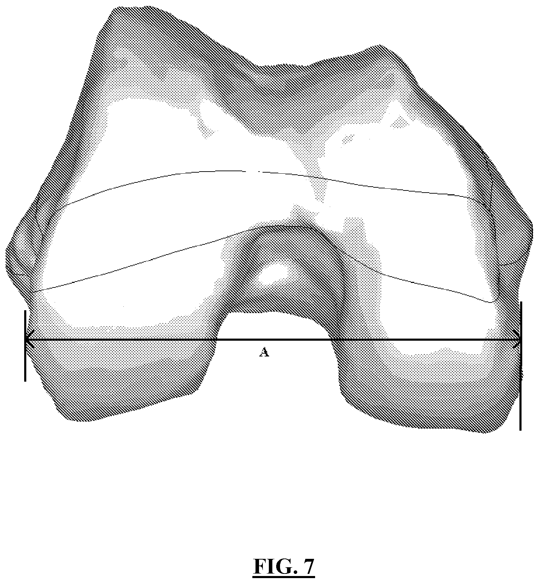

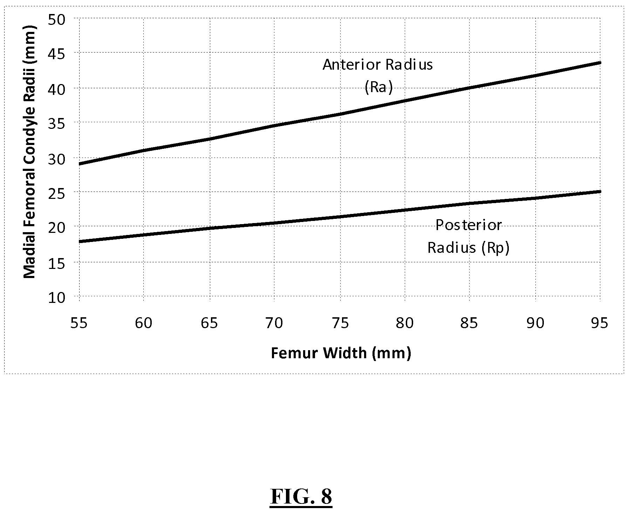

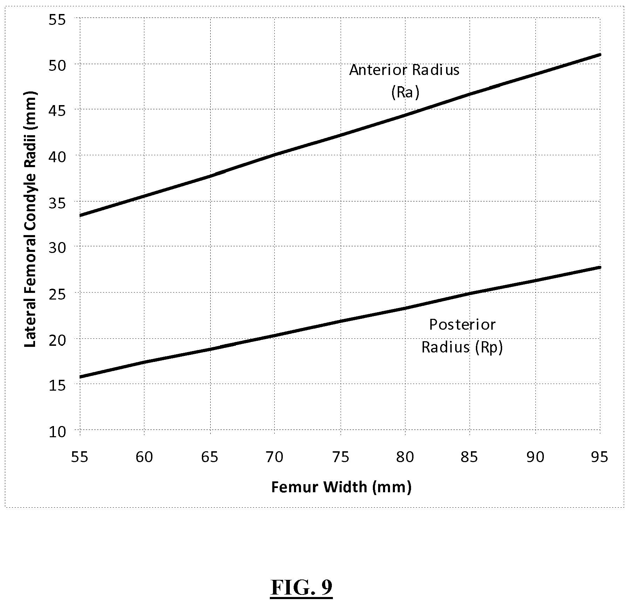

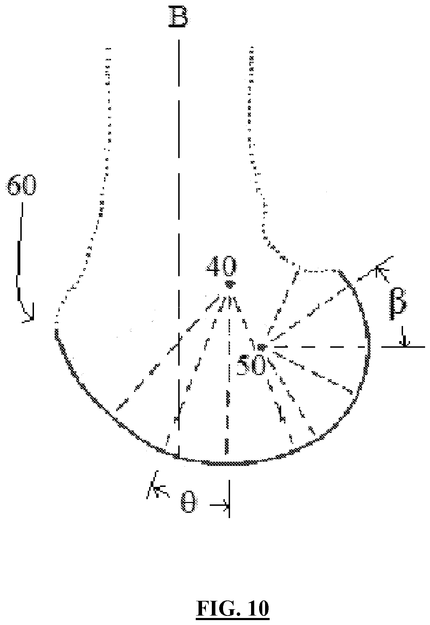

As can be seen from FIG. 6, various features of the femur and the femoral surfaces can be measured, including the various condylar widths, angles, and/or depths. In an exemplary embodiment, the width A of the distal femur at its widest point on the load bearing surface of the femur can be measured, as shown in FIG. 7. The width information can then be utilized to determine one or more exemplary features of the condylar surface of the femur. For example, FIGs. 8 and 9 graphically depict the result of an analysis of joints across a general population group, which has determined that there is a relationship between the width of the femur and the radii of the J-curves of the femoral condyles. See "Knee Morphology as a Guide to Knee Replacement," by Mensch and Amstutz, Clin Orthop Relat Res., 1975 Oct; (112):231-41. The measured femoral width is entered into the graphs of FIGs. 8 and 9, which reveal the derived anterior and posterior radii of the medial femoral condyle (FIG. 8) and the lateral femoral condyle (FIG. 9). An estimated shape for each of the femoral condyles can then be calculated using the previously determined values of the anterior radius and posterior radius on a generic condyle model 60. In the embodiment shown on FIG. 10, the anterior arc is fitted so that the anterior arc center 40 is positioned at a location wherein the lowest point of the femoral condyle is between 5 and 7 o'clock relative to the anterior arc center 40, and the posterior arc is positioned at a location wherein the furthest posterior point of the condyle is between 2 and 4 o'clock relative to the posterior arc center 50. In alternative embodiments, the anterior radius could be fitted to the contour of the extended knee to approximately 45° of knee flexion and the posterior radius could be fitted to approximately 120° of knee flexion. Placement of the radii can vary, although it is desirous that the two arcs defined by the radii meet smoothly and/or are relatively parallel at their meeting points, and one skilled in the art can position the arcs in various locations. Alternatively, the anterior and posterior arcs can be placed and modeled using measured dimensions of the patient's femoral anatomy and/or modeled on an actual and/or morphed electronic image of the imaged anatomy.

The results of the derived or estimated condylar surface shapes using indirect anatomical information can then be compared to the measured condylar surfaces of the patient's femur (or surfaces created using the direct measurement data on the condyle surface profile), and differences and/or deviations assessed. In a similar manner, surgical plans and/or surgical tools for preparation and implantation of a derived joint replacement implant (i.e., an implant designed to repair the derived condylar surface shapes) can be compared to the surgical plans/tools for the measured condylar surface shapes, and differences and/or deviations assessed. Where little or no differences exist, the system may simply utilize the derived and/or measured shapes (alone or through a combination of the two shapes) to create a surgical plan, tools, and implant design. However, where significant differences exist in the surface shapes, the proposed implant, and/or the proposed surgical plan, the system may identify such differences and alert the designer or other operator.

In various embodiments, the system may autonomously choose to utilize the derived surface shape in the design of the proposed joint replacement implant component(s) and surgical plan/tools, with the measured anatomical information utilized for planning of surgical cuts and surgical tool design (and for the internal bone-facing surfaces of the implant, if desired), as well as the placement of the derived implant components.

In one exemplary embodiment, a measurement of the femoral width A of a patient's femur (as per FIG. 7) is approximately 81 mm. This information is then cross-referenced using the graph of FIG. 8 to reveal derived anterior and posterior radii (for the medial condyle) of 37.5 mm and 22 mm, respectively. These radius values are then modeled on a generic femoral model (or on a model of the femoral head incorporating actual measured dimensional values, if desired) to construct a derived shape for the medial condylar articulating surface. The femoral width measurement is then cross-referenced using the graph of FIG. 9 to reveal derived anterior and posterior radii (for the lateral condyle) of 43.4 mm and 22.5 mm, respectively. Arcs with these radius values are then modeled on a generic femoral model (or on a model of the femoral head incorporating actual measured dimensional values, if desired) to construct a derived shape for the lateral condylar articulating surface. In the present embodiment, the arcs are positioned such that the anterior arc is positioned at a location wherein the lowest point of the femoral condyle is between 5 and 7 o'clock relative to the anterior arc center 40, and the posterior arc is positioned at a location wherein the furthest posterior point of the condyle is between 2 and 4 o'clock relative to the posterior arc center 50 (see FIG. 10).

In the described embodiment, the patient's actual medial condylar measurements revealed a medial condylar surface having anterior and posterior radii of 37 mm and 25 mm, respectively, which correlated relatively well with the derived values of 37.5 mm and 22 mm. However, the patient's actual lateral condylar measurements revealed a lateral condylar surface having anterior and posterior radii of 38.8 mm and 19.7 mm, respectively, which did not correlate well with the derived values of 43.4 mm and 22.5 mm. In such a case, it was highly likely that degradation of the patient's lateral condyle resulted in significant bone loss which was not readily apparent before the comparison step. In such a case, the joint replacement implant might have been designed to replicate the current patient anatomy, which might not be optimal for the patient. With the additional information provided by the various current embodiments, a more optimal and anatomically correct implant can be designed for the patient, with commensurately better outcomes for the patient.

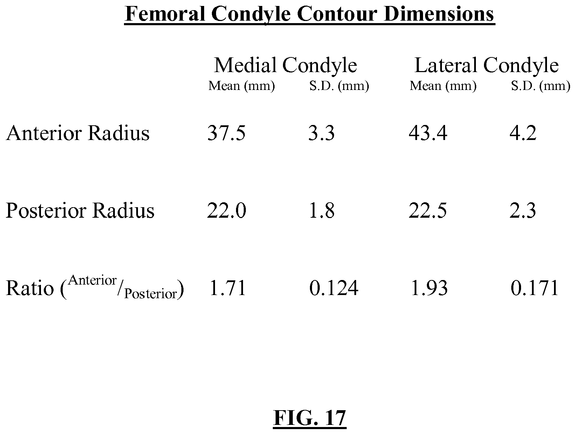

If desired, additional verification steps could be utilized to verify that the derived condylar features are appropriate to the patient model. For example, the ratio of the derived anterior and posterior radii (for the lateral condyle) would be 38.8 divided by 19.7, resulting in a ratio of 1.96 which, in this example, falls within an anticipated ratio (from the database) of 1.93 with a standard deviation of 0.171, indicating acceptable values as taken from the relevant database (see FIG. 17).

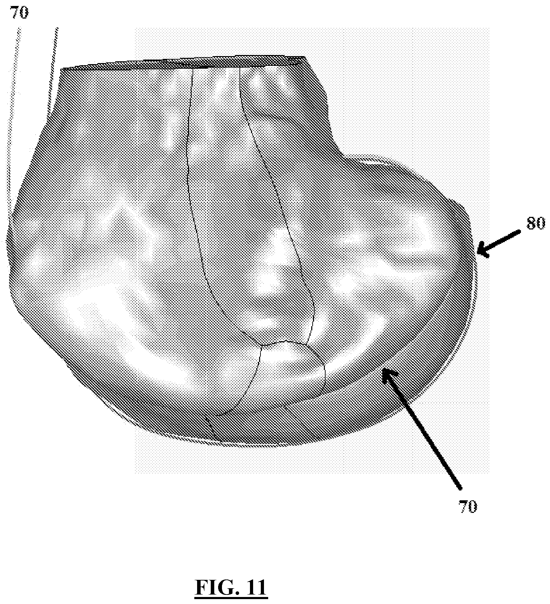

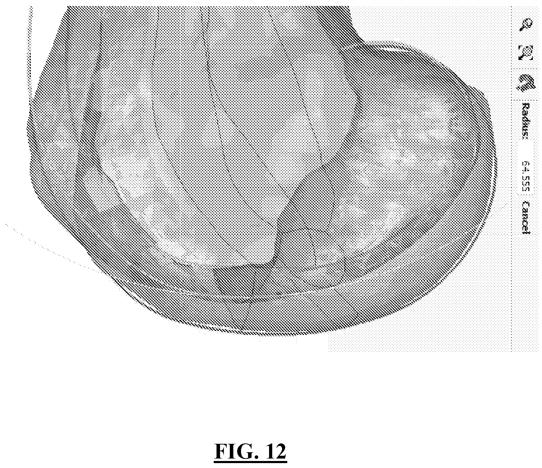

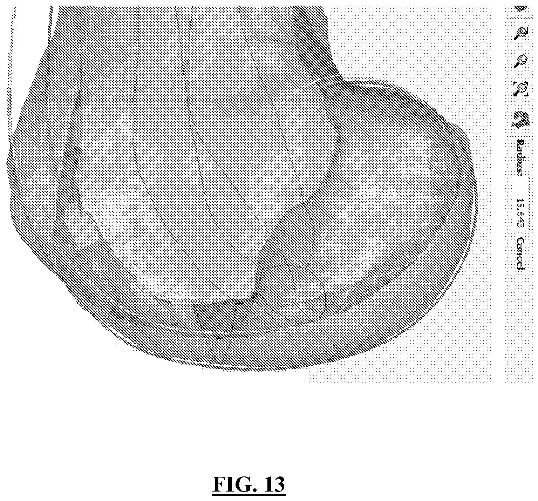

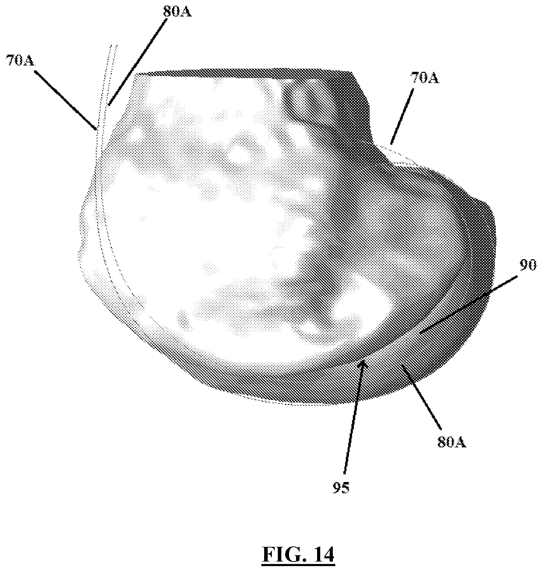

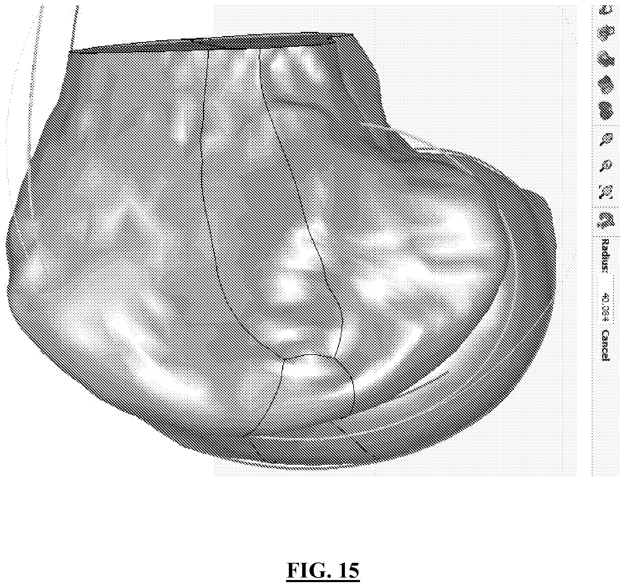

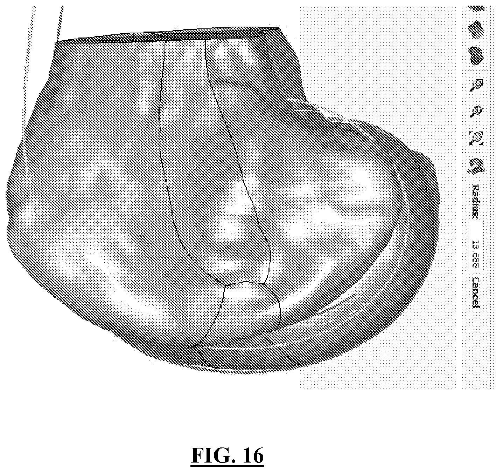

FIGs. 11 through 16 depict a representative outcome of J-curve derivation using the two concurrent methods as described herein. In FIGs. 11 through 13, the lateral and medial J-curves 70 and 80 are derived using actual measured dimensions of the condylar surfaces, and shown overlain on the actual femoral image. As seen in these figures, the lateral J-curve 70 generally follows the articular surfaces of the femoral condyle image, with anterior and posterior radii of 64.555 mm and 15.642 mm, respectively. FIGs. 14 through 16 depict the lateral and medial J-curves 70A and 80A derived in a method using a measured width of the femoral head, and then overlain onto a similar femoral image as used in FIG. 11, with the anterior and posterior radii of the lateral condyle estimated at 40.084 mm and 18.686 mm, respectively. As best seen in FIG. 14, the derived lateral condylar J-curve 70A departs from the actual articular surface 95 of the lateral condyle, with a gap 90 between the curve 70A and the actual articular surface 95. This gap 90 is a potential indication that the articular surface 95 has worn down or otherwise deformed, possibly through disease and/or patient use, and thus a desired joint replacement design could be designed and/or selected to account for the missing material (as shown by estimated surface 70A) to emulate a more healthy condition of the joint.

In various embodiments, it may be desirous to utilize the derived condylar articular surface contour measurements in designing and/or selecting the implant components. Alternatively, the variation may indicate that the anatomy is unsuitable for joint replacement of various types, and/or may be better suited for other surgical repairs or restorations.

### DERIVATION OF CONDYLAR WIDTH RELATIVE TO FEMORAL WIDTH

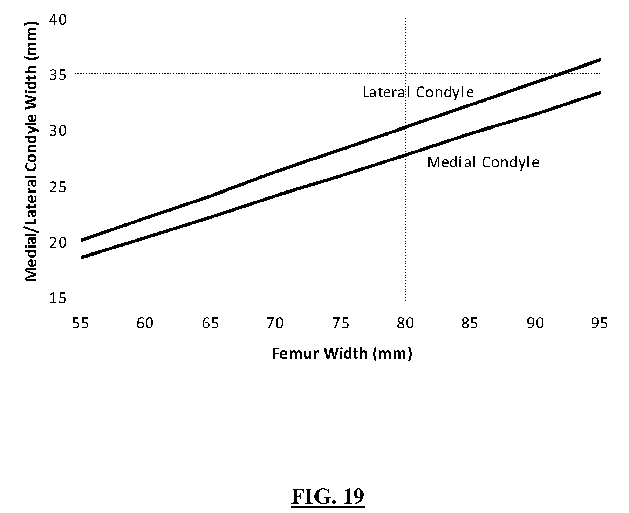

In another exemplary embodiment, a correlation between the femur width and the width of the medial and lateral condyles of a knee joint was obtained experimentally from a group of patient and cadaveric measurements, which is graphically depicted in FIG. 19. Accordingly, where one or both of the femoral condyles are heavily diseased, damaged, or otherwise malformed, the femoral width may be used to derive one or both of the respective condylar widths in a healthy, non-damaged, and/or otherwise more desirable state. This correlation information may similarly be utilized to improve an implant design, as well as to verify and/or validate that a given implant design meets or exceeds a given design threshold, which could include potentially "flagging" or otherwise highlighting design concerns to the program and/or the designer.

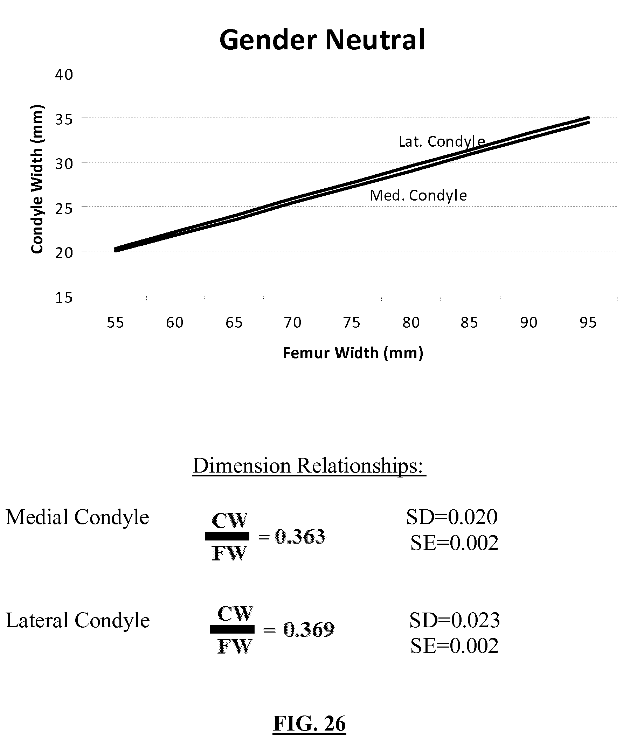

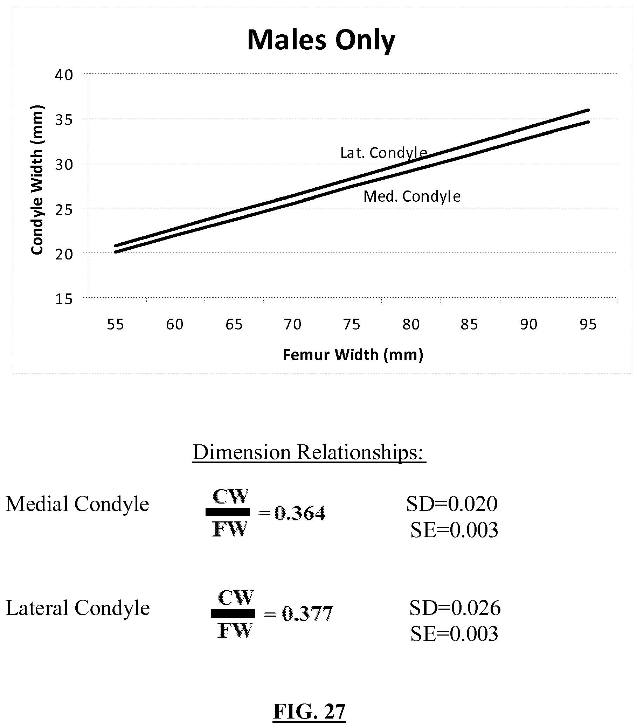

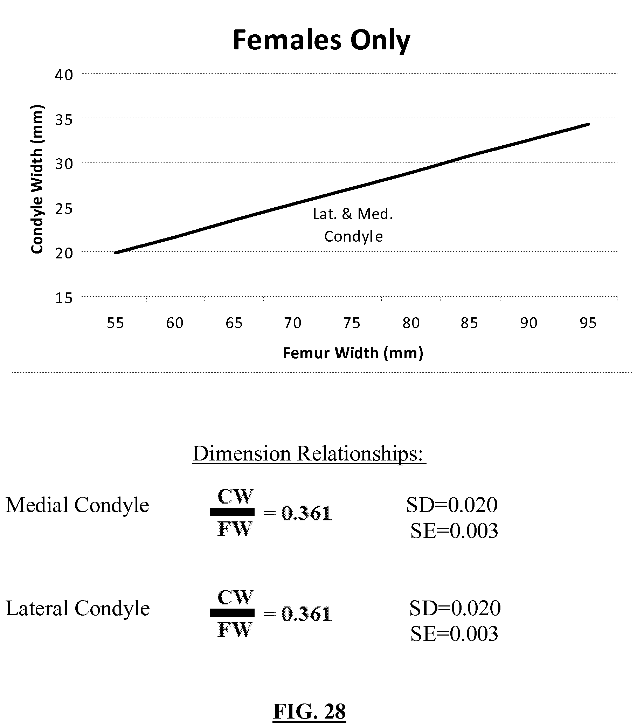

FIGs. 26 through 28 depict another set of correlations between the widths of the medial and lateral condyles and the width of a patient's femur, obtained experimentally from another group of patient and/or cadaveric measurements. These figures include gender-specific correlations (and a gender neutral correlation in FIG. 26) between the femoral width of a knee and the respective derived condylar width of both men and women. As with the previous correlation, where a femoral width is known or can be derived, this information can be utilized to derive the widths of one or both of the condyles in a healthy and/or non-damaged state. This correlation information may be similarly utilized to verify and/or validate that a given implant design meets or exceeds a given design threshold, which could include potentially "flagging" or otherwise highlighting design concerns to the program and/or the designer.

### ADDITIONAL DERIVATION OF CONDYLAR WIDTHS

An additional correlation that can be utilized from the graph of FIG. 19 would be a correlation between the widths of the condyles with respect to each other. In general, the graph reveals the width of the lateral condyle is greater than the width of the medial condyle, with specific values obtainable from the graph. Accordingly, where one or both of the femoral condyles are heavily diseased, damaged or otherwise malformed, the width of a first condyle may be used to derive a width of the opposing condyle in a healthy and/or non-damaged state. This correlation information may similarly be utilized to verify and/or validate that a given implant design meets or exceeds a given design threshold, which could include potentially "flagging" or otherwise highlighting design concerns to the program and/or the designer.

### DERIVATION OF FEMORAL CONDYLE DEPTH/LENGTH FROM FEMORAL WIDTH

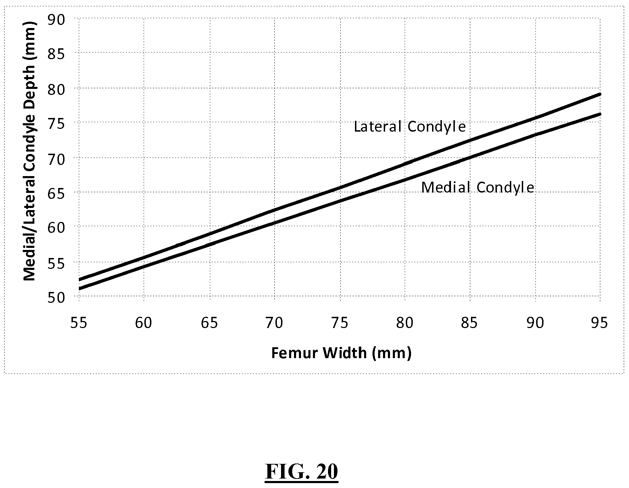

In another exemplary embodiment, a correlation between the depths (or lengths) of the medial and lateral condyles and the width of the patient's femur was obtained experimentally from a group of patient and cadaveric measurements. This analysis, best seen in FIG. 20, reveals that the lateral condyle depth can be approximately 3mm smaller than the medial condyle depth, with even greater depth differences seen in knees of larger sizes (and less in smaller knees). Accordingly, where a femoral width is known or can be derived, this information can be utilized to derive the depth of one or both of the condyles in a healthy and/or non-damaged state. This correlation information may be similarly utilized to verify and/or validate that a given implant design meets or exceeds a given design threshold, which could include potentially "flagging" or otherwise highlighting design concerns to the program and/or the designer.

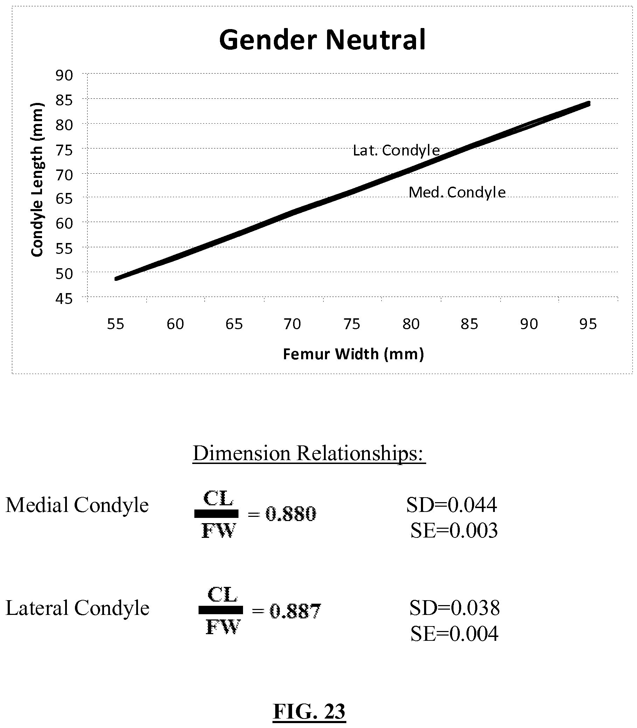

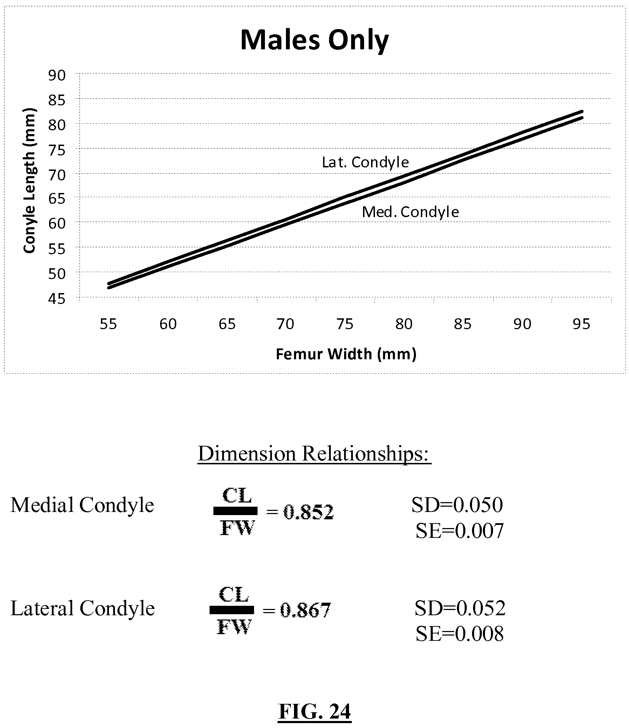

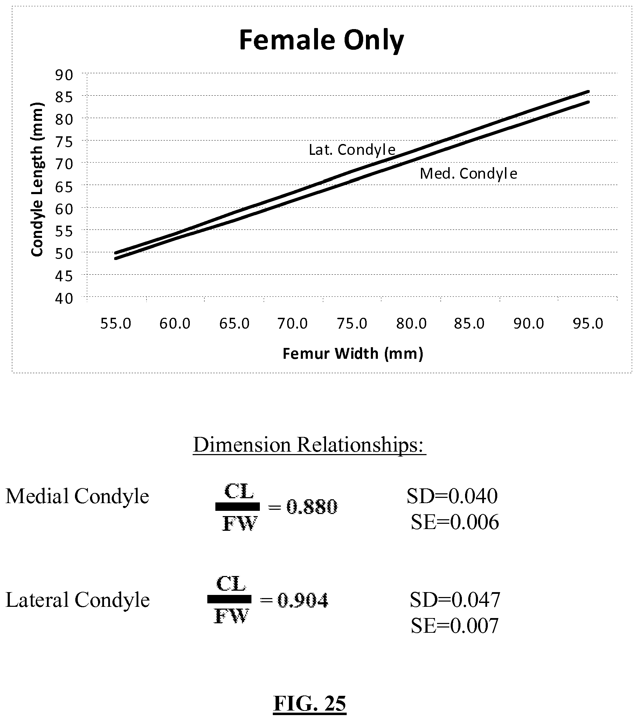

FIGs. 23 through 25 depict another set of correlations between the lengths (or depths) of the medial and lateral condyles and the width of a patient's femur, obtained experimentally from another group of patient and/or cadaveric measurements. These figures include gender-specific correlations (and a gender neutral correlation in FIG. 23) between the femoral width of a knee and the respective derived condylar length of both men and women. As with the previous correlation, where a femoral width is known or can be derived, this information can be utilized to derive the lengths of one or both of the condyles in a healthy and/or non-damaged state. This correlation information may be similarly utilized to verify and/or validate that a given implant design meets or exceeds a given design threshold, which could include potentially "flagging" or otherwise highlighting design concerns to the program and/or the designer.

### DERIVATION OF NOTCH WIDTH FROM FEMORAL WIDTH

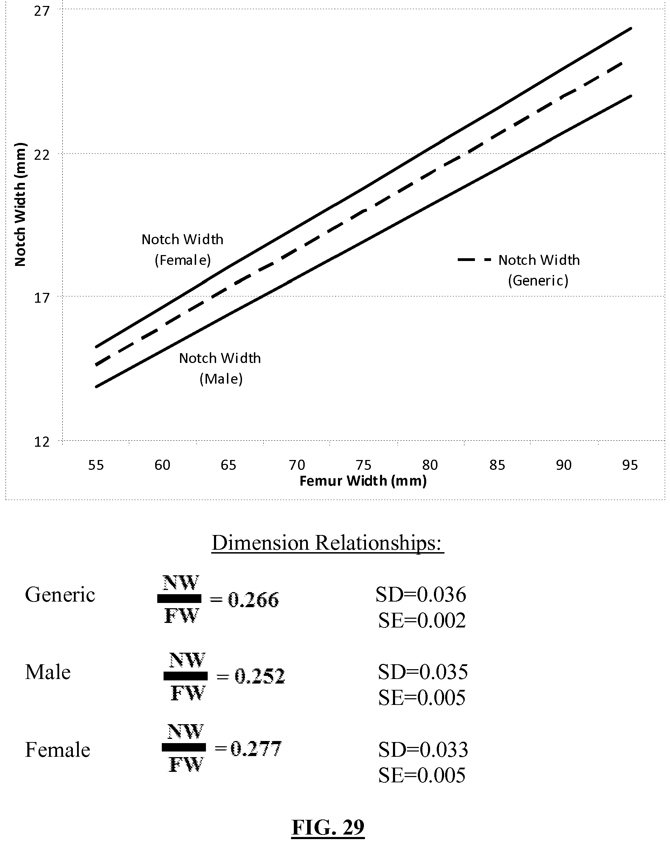

In another exemplary embodiment, a correlation between the width of a femoral notch and the width of the patient's femur was obtained experimentally from a group of patient and cadaveric measurements. FIG. 29 includes gender-specific and gender-neutral correlations between the femur width of a knee and the respective femoral notch width of both men and women. Accordingly, where a femoral width is known or can be derived, this information can be utilized to derive the widths of the femoral notch in a healthy and/or non-damaged state. This correlation information may be similarly utilized to verify and/or validate that a given implant design meets or exceeds a given design threshold, which could include potentially "flagging" or otherwise highlighting design concerns to the program and/or the designer.

### DERIVATION OF KNEE SIZE RELATIVE TO HEIGHT

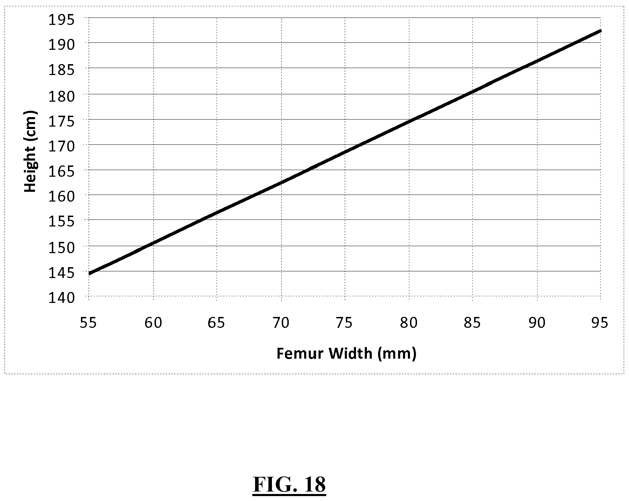

In another exemplary embodiment, a derived femur width can be determined from a height measurement of the patient. FIG. 18 depicts an exemplary correlation between patient height and femoral width obtained experimentally from a group of patient and cadaveric measurements. The derived femoral width can subsequently be used to derive various anatomical information regarding the patient, including derived contours for medial and lateral condylar J-Curves, as previously described.

In general, an individual's height decreases with advancing age. Because patient height changes can result from a variety of factors, including degeneration and/or deterioration of the spine as well as various other joints (i.e., thinning and/or compression of spinal discs, vertebral fractures, hip degeneration, etc.), it may be desirous to obtain one or more historical records of a patient's height, such as during the patent's mid-twenties, and use this historical height information to derive the anatomical information related to the knee anatomy. The derived information can then be utilized in the various methods described herein to assist with implant design and/or selection as well as verify proper implant design.

In one exemplary embodiment, a 65 year old patient has a measured height of 175 mm (approximately 5'-9"). Historical records such as driver's license information or other documentation (e.g., a DD-214 from military discharge at an earlier age) reveals the patient was 185.5 mm tall (approximately 6'-1") at age 25. Using the information from a relevant database, such as provided in FIG. 18, a derived femoral width of 80 mm (for the patient's height at 60 years old) and/or 89 mm (for the patient's height at 25 years old) is obtained. Either or both of these values may be compared to the measured width of the patient's femur, and deviations identified. Moreover, either or both of these values may be further utilized to derive other anatomical data and/or surface characteristics of the patient's knee for design/selection of a knee replacement and/or verification of implant characteristics obtained through direct measurement of the patient's anatomy.

### DERIVATION OF FEMORAL CONDYLE SHAPES FROM TIBIA WIDTH

In another exemplary embodiment, an anatomical relationship between the widths of the tibia and femur can be utilized to derive other anatomical characteristics of the knee joint. For example, where the femur is heavily diseased, damaged, or otherwise malformed, the width of the tibia may be used to derive the width of the femur in a healthy and/or non-damaged state. This derived femoral width can then be utilized to derive the various shapes of the articulating surfaces of the medial and/or lateral condyles, as previously described.

In one exemplary embodiment, a correlation between femoral and tibial width was obtained experimentally from a group of patient and cadaveric measurements, resulting in a direct correlation (1:1) between the measured and derived widths of the femur and tibia.

In a similar manner, a width of a healthy femur can be used to derive a width of a diseased, damaged, and/or otherwise malformed tibial width.

If desired, the derived value(s) may then be compared to the measured width of the patient's anatomy, and deviations identified. Moreover, the derived values may be further utilized to derive other anatomical data and/or surface characteristics of the patient's knee for design/selection of a knee replacement and/or verification of implant characteristics obtained through direct measurement of the patient's anatomy.

### DERIVATION OF ANTERIOR AND/OR POSTERIOR CONDYLE RADII, SHAPES, AND/OR SIZES

In another exemplary embodiment, anatomical relationships between the anterior and/or posterior radii of the medial and lateral condyles can be utilized to derive anatomical characteristics of the knee joint.

### POSTERIOR RADII RELATIONSHIP:

In one exemplary embodiment, a correlation between the posterior radii of the medial and lateral condyles of a knee joint was obtained experimentally from a group of patient and cadaveric measurements, resulting in a direct correlation (1:1) between the measured and derived posterior radii. Accordingly, where a first condyle of the femur is heavily diseased, damaged, or otherwise malformed, the posterior radii of the opposing (second) condyle of the joint may be used to derive the posterior radii of the first condyle of the femur in a healthy and/or non-damaged state. This correlation information may similarly be utilized to verify and/or validate that a given implant design meets or exceeds a given design threshold, which could include potentially "flagging" or otherwise highlighting design concerns to the program and/or the designer.

### ANTERIOR RADII RELATIONSHIP:

In another exemplary embodiment, a correlation between the anterior radii of the medial and lateral condyles of a knee joint was obtained experimentally from a group of patient and cadaveric measurements. This analysis revealed the anterior radius of the lateral femoral condyle averaged 5.9 mm larger than the anterior radius of the medial femoral condyle of the same knee. Accordingly, where a first condyle of the femur is heavily diseased, damaged, or otherwise malformed, the anterior radii of the opposing (second) condyle of the joint may be used to derive the anterior radii of the first condyle of the femur in a healthy and/or non-damaged state. This correlation information may similarly be utilized to verify and/or validate that a given implant design meets or exceeds a given design threshold, which could include potentially "flagging" or otherwise highlighting design concerns to the program and/or the designer.

### ANTERIOR RADII TO POSTERIOR RADII RELATIONSHIP:

In another exemplary embodiment, a correlation between the anterior and posterior radii of the individual medial and lateral condyles was obtained experimentally from a group of patient and cadaveric measurements. This analysis revealed an anterior to posterior radii ratio of 1.7:1 for the medial condyle, and an anterior to posterior radii ratio range of 1.9:1 to 2:1 for the lateral condyle. Accordingly, where an anterior or posterior portion of a single condyle of the femur is heavily diseased, damaged, or otherwise malformed, the opposing radii (i.e., anterior or posterior) of the same condyle may be used to derive an appropriate radii and condylar surface portion in a healthy and/or non-damaged state. This correlation information may be similarly utilized to verify and/or validate that a given implant design meets or exceeds a given design threshold, which could include potentially "flagging" or otherwise highlighting design concerns to the program and/or the designer.

### CONDYLAR DEPTH RELATIONSHIP:

An additional correlation that can be utilized from the graph of FIG. 20 is a correlation between the respective depths of the medial and lateral condyles. The analysis of the experimental data revealed that the lateral condyle depth is approximately 3mm smaller than the medial condyle depth, with even greater than 3mm depth differences in knees of larger sizes (and lesser depths differences for smaller knees). Accordingly, where a first condyle of the femur is heavily diseased, damaged, or otherwise malformed, the opposing condyle of the same knee joint may be used to derive an appropriate and/or minimum/maximum depth of the first condyle in a healthy and/or non-damaged state. This correlation information may be similarly utilized to verify and/or validate that a given implant design meets or exceeds a given design threshold, which could include potentially "flagging" or otherwise highlighting design concerns to the program and/or the designer.

### DERIVATION OF TIBIAL PLATEAU DEPTHS FROM FEMORAL WIDTH

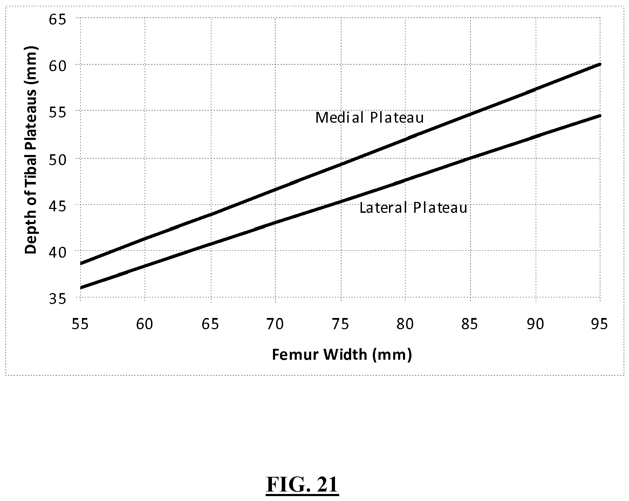

In another exemplary embodiment, a correlation between the femoral width and the depths of the medial and lateral tibial plateaus was obtained experimentally from a group of patient and cadaveric measurements, as best seen in FIG. 21. Accordingly, where one or both of the tibial plateaus are heavily diseased, damaged, or otherwise malformed, the femoral width of the joint may be used to derive the depths of the medial and lateral tibial plateaus in a healthy and/or non-damaged state. This correlation information may similarly be utilized to verify and/or validate that a given implant design meets or exceeds a given design threshold, which could include potentially "flagging" or otherwise highlighting design concerns to the program and/or the designer.

### DERIVATION OF PATELLAR DIMENSIONS FROM FEMORAL WIDTH

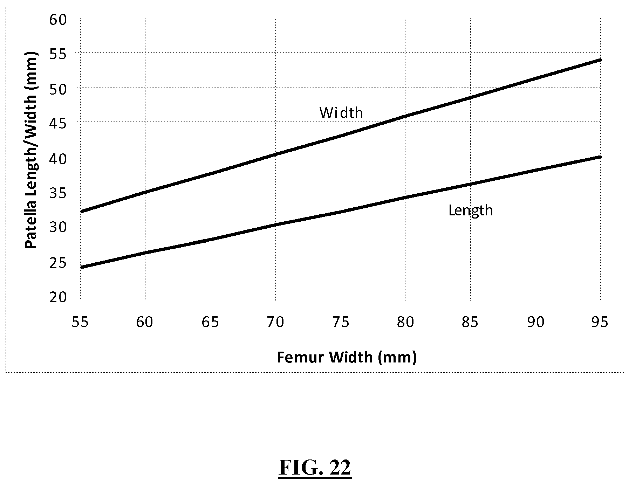

In another exemplary embodiment, a correlation between the femoral width and the length and/or width of the patella was obtained experimentally from a group of patient and cadaveric measurements, as best seen in FIG. 22. Accordingly, where one or more of the patellar surfaces are heavily diseased, damaged, or otherwise malformed, the femoral width of the joint may be used to derive the length and/or width of the patella in a healthy and/or non-damaged state. This correlation information may similarly be utilized to verify and/or validate that a given implant design meets or exceeds a given design threshold, which could include potentially "flagging" or otherwise highlighting design concerns to the program and/or the designer.

### DERIVATION OF FEMORAL WIDTH

In some instances, various portions of the femur of a patient may be diseased, damaged, or otherwise malformed such that the femoral width is difficult to measure and/or is substantially different from the patient's healthy femoral width. In such instances, various embodiments may include deriving the femoral width based on one or more measurements of other anatomical dimensions of the patient (including, e.g., lateral femoral condyle depth/length, medial femoral condyle depth/length, notch width, lateral condyle width, medial condyle width, notch width, or height) and corresponding anatomical relationship correlations, which relate the measured anatomical feature(s) to the femoral width, such as, for example, those illustrated in FIGs. 8, 9, 18-29. In some embodiments, one or more particular anatomical dimensions may be selected to be measured based on one or more of the estimated health of tissue that underlies the dimension(s), the determined or estimated strength of the corresponding anatomical relationship correlation(s) in accurately predicting the femoral width, and the ease of accurately measuring the particular dimension. By way of example, in some embodiments the lateral femoral condyle depth/length and/or medial femoral condyle depth/length may be selected as the anatomical dimension(s) from which to derive the femoral width because such anatomical dimension(s) may be known to be the most accurate dimensions for predicting femoral width. Additionally, in some embodiments, once the femoral width has been derived, this value may be utilized to derive other anatomical dimensions (not used in its derivation), such as, for example, anterior and/or posterior radii of the medial and/or lateral condyles.

Furthermore, in some embodiments, multiple predicted femoral widths may be derived based on measurements of multiple anatomical dimensions and use of the corresponding anatomical relationship correlations, and the multiple predicted femoral widths may be averaged to determine a final prediction of the femoral width. For example, in some embodiments, as many anatomical dimensions (e.g., lateral femoral condyle depth/length, medial femoral condyle depth/length, notch width, lateral condyle width, medial condyle width, notch width, and height) as possible may be measured and utilized along with each respective anatomical dimension's corresponding anatomical relationship correlation, as discussed above, to derive respective predicted femoral widths. Each predicted femoral width may vary slightly from the others due to various causes, such as, for example, the accuracy of the measurement of the respective anatomical dimension, the accuracy of the respective anatomical relationship correlations in predicting the femoral width, the effect of disease and/or damage on anatomy underlying the respective dimensions, etc. The multiple predicted femoral widths may averaged to determine a final predicted femoral width.

### CREATION AND/OR MODIFICATION OF ANATOMICAL RELATIONSHIP CORRELATIONS AND DATABASES

In various embodiments, historical and/or experimental data, libraries, and/or databases can be created, utilized, modified, and/or otherwise manipulated to improve the design and/or selection of joint replacement implants, surgical tools, and plans, including the various systems and methods described herein.

The various anatomical relationship correlations described and referenced herein can be obtained from observations and assessments of anatomical features from a number of selected individuals. The selection criteria can vary, and include healthy and/or diseased/damaged individuals from selected age groups, races, ethnicities, occupations, abilities, heights, weights, activity levels, disease states, pain tolerance, etc. Selection criteria can also include outcomes data from previous joint replacement procedures. Additional embodiments can include the creation of various databases incorporating data regarding patient anatomical structures and studies, derived anatomical features, implant features, surgical tool features, surgical plans and surgical results, outcomes and long-term results. The database or databases can be cross-referenced, analyzed and or queried, and various correlations between data in an individual database as well as between databases can be identified and utilized as necessary. For example, information relevant to the surgical joint replacement procedures for one or more patients (including measured anatomical features, derived anatomical features, implant designs, surgical tool designs, surgical plans, surgical notes, procedural specifics (i.e., surgical time, type and quantity of anesthetic, units of whole blood used, etc.) physician's operative notes, range-of-motion studies, post-operative data, outcomes, and/or long-term follow-up data) can be entered into one or more databases. The database(s) can then be queried and/or analyzed to identify and/or determine correlations that exist between the various data within and/or between the database(s). As additional data becomes available, it can be added to the database and utilized as described herein.

With a sufficient amount of relevant data in the database (i.e., a large number of patient and/or implant records), as well as proper evaluation and selection of relevant data group(s), accurate and reliable anatomical correlations can be determined and utilized to improve implant, surgical tool, and surgical plan designs. Similarly, the use and analysis of a sufficient amount of outcomes data can potentially provide accurate and reliable estimation of the potent success/failure of a given implant, surgical tool, and/or surgical plan design as it relates to measured and/or derived anatomical data on a given patient.

In one embodiment, anatomical information is acquired for a potential patient. The physician and/or designer then assigns one or more selection criteria based on a patient's individual or group characteristics. This process is repeated for multiple patients, and then one or more anatomical relationship correlations are derived from analysis of anatomical and outcome records from select series of individuals meeting the selection criteria. If desired, different database subjects and/or groups of subject may be identified as relevant to each anatomical feature/anatomical relationship and/or each selection criteria, and alternative and multiple selection criteria may be assessed for a given anatomical feature. The anatomical relationship correlations can then be utilized to derive other anatomical data and/or surface characteristics of the patient's knee through the various methods described herein. Derived anatomical data can then be compared to actual anatomical measurements, as previously described, as well as used for design/selection of a knee replacement and/or verification of implant characteristics obtained through direct measurement of the patient's anatomy.

The use of such databases allows for near real-time modification and/or manipulation of anatomical data and correlation information, as well as increasing the accuracy and/or specificity of the various anatomical relationship correlations. In a similar manner, data and databases of unsuccessful patient outcomes and/or unusual patient anatomy may be used and compared (as described herein) as verification or some other guiding features for the designer to avoid.

### CREATION OF VIRTUAL AND/OR PHYSICAL LIBRARIES OF IMPLANT DESIGNS

In various embodiments, the systems and methods described herein could include the use of various anatomical relationship correlations, including those described herein, as well as others determined experimentally and/or obtained from other sources (e.g., database queries, literature reviews, etc.), to create and/or develop pre-designed (i.e., virtual) and/or pre-manufactured implant libraries as well as associated libraries of surgical tools and surgical procedures. Such libraries could include virtual libraries of implant and/or surgical tool designs, with various designs having predetermined dimensions and/or shapes to accommodate various patient anatomy. Other designs in the virtual library could comprise some predetermined dimensions and/or shapes in combination with other dimensions and/or shapes that can be variable and/or otherwise modified or manipulated using patient-specific anatomical data, derived anatomical features and/or various anatomical relationship correlations. As part of the design and/or selection process, the virtual library could be queried and one or more virtual designs chosen (and further modified, if desired) for subsequent manufacturing and patient implantation of the physical implant. Such manufacturing could include "just-in-time" type manufacturing and inventory methods.

If desired, the library could include a physical inventory of a number of implants of different shapes and/or sizes designed, at least in part, using the various system and methods described herein. Such physical implants could include stockpiles of various pre-determined sizes and shapes of implants, which could be designed using the various systems and methods described herein, including scaling of various implant shapes and/or sizes. Physical implant libraries could include a few implants of a few sizes/shapes, or could include numerous implants of a wide variety of sizes/shapes. If desired, the library could include one or more series of pre-manufactured implant "blanks" having various pre-determined dimensions and/or shapes, with a virtual library query identifying one or more chosen virtual designs that somewhat approximate the chosen blank(s), with the individual blank or blanks selected for further processing (i.e., material removal and/or addition, further shaping of surfaces, assembly or disassembly of various implant components of different shapes and/or sizes, etc.) to more closely approximate the chosen virtual design for ultimate implantation into the patient. Such manufacturing could include "just-in-time" type manufacturing and inventory methods.

Similar approaches as described herein could be used for creating surgical tools and/or various associated surgical procedures.

It is to be understood that the features of the various embodiments described herein are not mutually exclusive and may exist in various combinations and permutations.

## Claims

1. A method of making an implant component for a joint of a patient, comprising:
obtaining an anatomical dimension associated with the patient;
deriving information regarding an anatomical feature of the joint based, at least in part, on the anatomical dimension and an anatomical relationship correlation;
designing an implant component based, at least in part, on the derived information regarding the anatomical feature;
measuring the anatomical feature to determine measured information regarding the anatomical feature; comparing the measured information regarding the anatomical feature to the derived information regarding the anatomical feature;
wherein if there is substantially no difference between the measured information regarding the anatomical feature and the derived information regarding the anatomical feature, designing the implant component is based, at least in part, on at least one of the measured information regarding the anatomical feature and the derived information regarding the anatomical feature, and if the measured information regarding the anatomical feature is substantially different from the derived information regarding the anatomical feature, the method further comprises providing an alert to the designer of the implant.

2. The method of claim 1, wherein the joint is a knee joint, wherein the anatomical dimension comprises a femur width, a patella width, a patella length, a condyle length, a condyle width, or a femoral notch width of the knee joint,
or
the method of claim 1, wherein the obtaining an anatomical dimension comprises measuring the anatomical dimension directly from an image of the joint.

3. The method of claim 1, wherein the joint is a knee joint, wherein the anatomical feature comprises at least one of a lateral femoral condyle of the knee joint, a medial femoral condyle of the knee joint, and a patella of the knee joint.

4. The method of claim 1, wherein the joint is a knee joint, wherein the derived information regarding the anatomical feature of the knee joint comprises at least one of an anterior radius of a femoral condyle, a posterior radius of a femoral condyle, a femur width, a patella width, a patella length, a condyle length, a condyle width, or a femoral notch width of the knee joint.

5. The method of claim 1, wherein the anatomical dimension comprises the patient's height.

6. The method of claim 1, wherein the anatomical relationship correlation relates the anatomical dimension to the derived information regarding the anatomical feature.

7. The method of claim 1, further comprising selecting the anatomical relationship correlation from a database, optionally wherein the selecting the anatomical relationship correlation is based, at least in part, on patient-specific information, further optionally wherein the patient-specific information comprises at least one of the patient's age, weight, gender, and race.

8. The method of claim 1, wherein the joint is a knee joint, the method further comprising using the derived information to estimate at least a portion of a condyle or the knee joint.

## Patentansprüche

1. Ein Verfahren zum Herstellen einer Implantatkomponente für ein Gelenk eines Patienten, umfassend:
Erhalten einer anatomischen Dimension, die dem Patienten zugeordnet ist;
Ableiten von Informationen bezüglich eines anatomischen Merkmals des Gelenks, zumindest teilweise basierend auf der anatomischen Dimension und einer anatomischen Beziehungskorrelation;
Entwerfen einer Implantatkomponente, zumindest teilweise basierend auf abgeleiteten Informationen in Bezug auf das anatomische Merkmal;
Messen des anatomischen Merkmals, um gemessene Informationen bezüglich des anatomischen Merkmals zu bestimmen; Vergleichen der gemessenen Informationen bezüglich des anatomischen Merkmals mit den abgeleiteten Informationen bezüglich des anatomischen Merkmals;
wobei, wenn es im Wesentlichen keinen Unterschied zwischen den gemessenen Informationen bezüglich des anatomischen Merkmals und den abgeleiteten Informationen bezüglich des anatomischen Merkmals gibt, das Entwerfen der Implantatkomponente zumindest teilweise auf mindestens einer der gemessenen Informationen bezüglich des anatomischen Merkmals und den abgeleiteten Informationen bezüglich des anatomischen Merkmals basiert, und
wenn die gemessenen Informationen bezüglich des anatomischen Merkmals im Wesentlich unterschiedlich zu den abgeleiteten Informationen bezüglich des anatomischen Merkmals sind, das Verfahren ferner das Bereitstellen einer Warnung an den Konstrukteur des Implantats umfasst.

2. Das Verfahren nach Anspruch 1, wobei das Gelenk ein Kniegelenk ist, wobei die anatomische Dimension eine Femurbreite, eine Patellabreite, eine Patellalänge, eine Kondylenlänge, eine Kondylenbreite oder eine femorale Kerbbreite des Kniegelenks umfasst, oder
das Verfahren nach Anspruch 1, wobei das Erhalten einer anatomischen Dimension die Messung der anatomischen Dimension direkt von einem Bild des Gelenks umfasst.

3. Das Verfahren nach Anspruch 1, wobei das Gelenk ein Kniegelenk ist, wobei das anatomische Merkmal mindestens eines von einem lateralen Femurkondylus des Kniegelenks, einem medialen Femurkondylus des Kniegelenks und einer Patella des Kniegelenks umfasst.

4. Das Verfahren nach Anspruch 1, wobei das Gelenk ein Kniegelenk ist, wobei die abgeleiteten Informationen bezüglich des anatomischen Merkmals des Kniegelenks mindestens eines von einem Vorderradius eines Femurkondylus, einem Hinterradius eines Femurkondylus, einer Femurbreite, einer Patellabreite, einer Patellalänge, einer Kondylenlänge, einer Kondylenbreite oder einer femoralen Kerbbreite des Kniegelenks umfasst.

5. Das Verfahren nach Anspruch 1, wobei die anatomische Dimension die Größe des Patienten umfasst.

6. Das Verfahren nach Anspruch 1, wobei sich die anatomische Beziehungskorrelation auf die anatomische Dimension zu den abgeleiteten Informationen bezüglich des anatomischen Merkmals bezieht.

7. Das Verfahren nach Anspruch 1, ferner umfassend das Auswählen der anatomischen Beziehungskorrelation aus einer Datenbank, wobei optional das Auswählen der anatomischen Beziehungskorrelation zumindest teilweise auf patientenspezifischen Informationen basiert, wobei ferner optional die patientenspezifischen Informationen mindestens eines von dem Alter, dem Gewicht, dem Geschlecht und der Rasse des Patienten umfasst.

8. Das Verfahren nach Anspruch 1, wobei das Gelenk ein Kniegelenk ist, wobei das Verfahren ferner das Verwenden der abgeleiteten Informationen umfasst, um mindestens einen Teil eines Kondylus oder des Kniegelenks abzuschätzen.

## Revendications

1. Procédé de fabrication d'un composant d'implant pour une articulation d'un patient, comprenant :
l'obtention d'une dimension anatomique associée au patient ;
la dérivation d'informations concernant une caractéristique anatomique de l'articulation sur la base, au moins en partie, de la dimension anatomique et d'une corrélation de relations anatomiques ;
la conception d'un composant d'implant sur la base, au moins en partie, des informations dérivées concernant la caractéristique anatomique ;
la mesure de la caractéristique anatomique pour déterminer des informations mesurées concernant la caractéristique anatomique ; la comparaison des informations mesurées concernant la caractéristique anatomique aux informations dérivées concernant la caractéristique anatomique ;
dans lequel, s'il n'y a aucune différence significative entre les informations mesurées concernant la caractéristique anatomique et les informations dérivées concernant la caractéristique anatomique, la conception du composant d'implant est basée, au moins en partie, sur au moins l'une parmi les informations mesurées concernant la caractéristique anatomique et les informations dérivées concernant la caractéristique anatomique, et
si les informations mesurées concernant la caractéristique anatomique sont sensiblement différentes des informations dérivées concernant la caractéristique anatomique, le procédé comprend en outre la fourniture d'une alerte au concepteur de l'implant.

2. Procédé selon la revendication 1, dans lequel l'articulation est une articulation du genou, dans lequel la dimension anatomique comprend une largeur de fémur, une largeur de rotule, une longueur de rotule, une longueur de condyle, une largeur de condyle, ou une largeur d'entaille fémorale de l'articulation du genou,
ou
procédé selon la revendication 1, dans lequel l'obtention d'une dimension anatomique comprend la mesure de la dimension anatomique directement à partir d'une de l'articulation.

3. Procédé selon la revendication 1, dans lequel l'articulation est une articulation du genou, dans lequel la caractéristique anatomique comprend au moins l'un parmi un condyle fémoral latéral de l'articulation du genou, un condyle fémoral médian de l'articulation du genou, et une rotule de l'articulation du genou.

4. Procédé selon la revendication 1, dans lequel l'articulation est une articulation du genou, dans lequel les informations dérivées concernant la caractéristique anatomique de l'articulation du genou comprennent au moins l'un parmi un rayon antérieur d'un condyle fémoral, un rayon postérieur d'un condyle fémoral, une largeur de fémur, une largeur de rotule, une longueur de rotule, une longueur de condyle, une largeur de condyle, ou une largeur d'entaille fémorale de l'articulation du genou.

5. Procédé selon la revendication 1, dans lequel la dimension anatomique comprend la taille du patient.

6. Procédé selon la revendication 1, dans lequel la corrélation de relations anatomiques relie la dimension anatomique aux informations dérivées concernant la caractéristique anatomique.

7. Procédé selon la revendication 1, comprenant en outre la sélection de la corrélation de relations anatomiques à partir d'une base de données, facultativement dans lequel la sélection de la corrélation de relations anatomiques est basée, au moins en partie, sur des informations spécifiques au patient, facultativement dans lequel les informations spécifiques au patient comprennent en outre au moins l'un parmi l'âge, le poids, le sexe et la race du patient.

8. Procédé selon la revendication 1, dans lequel l'articulation est une articulation du genou, le procédé comprenant en outre l'utilisation des informations dérivées pour estimer au moins une partie d'un condyle ou de l'articulation du genou.
